(19) Europäisches Patentamt

European Patent Office

Office européen des brevets

(11) **EP 1 060 705 A1**

(12) **DEMANDE DE BREVET EUROPEEN**

(43) Date de publication:
**20.12.2000 Bulletin 2000/51**

(51) Int. Cl.$^7$: **A61B 5/0285**, A61B 5/021

(21) Numéro de dépôt: **00401432.0**

(22) Date de dépôt: **23.05.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **16.06.1999 FR 9907632**

(71) Demandeur: **Global Link Finance Al Maten (LB)**

(72) Inventeur: **Asmar, Roland**
**75016 Paris (FR)**

(74) Mandataire:
**Blot, Philippe Robert Emile et al
c/o Cabinet Lavoix,
2, place d'Estienne d'Orves
75441 Paris Cedex 09 (FR)**

(54) **Procédé de détermination de la vitesse d'une onde de pouls et d'estimation de la pression aortique**

(57) L'invention concerne un procédé de détermination du décalage temporel entre les instants de passage d'une même onde de pouls en deux points de mesure distincts ($M_1$, $M_2$) d'un réseau artériel d'un être vivant, comportant les étapes de :

a) placer un capteur de pression (14,16) à chaque point de mesure du réseau artériel ;
b) enregistrer l'évolution au cours du temps de l'onde de pression artérielle à chaque point de mesure ;
c) déduire des deux enregistrements ledit décalage temporel, caractérisé en ce que l'étape de déduction du décalage temporel comporte les étapes de :
c1) déterminer, pour un premier enregistrement, un premier instant ($t_1$) d'apparition de l'onde de pouls en identifiant le pied de l'onde de pouls,
c2) déterminer, pour le deuxième enregistrement, un deuxième instant ($t_2$) d'apparition de la même onde de pouls en identifiant, dans le deuxième enregistrement, l'apparition de la partie correspondante de l'onde de pouls,
c3) calculer le décalage temporel à partir des instants $t_1$ et $t_2$ déterminés sur les premier et deuxième enregistrements.

FIG.1

EP 1 060 705 A1

Printed by Xerox (UK) Business Services
2.16.7 (HRS)/3.6

**Description**

La présente invention concerne un procédé de détermination du décalage temporel entre les instants de passage d'une même onde de pouls en deux points de mesure distincts d'un réseau artériel d'un être vivant. Elle concerne en outre un procédé de détermination de la vitesse de propagation de l'onde de pouls entre ces deux points de mesure, à partir du décalage temporel déterminé.

Elle concerne enfin un procédé d'estimation de la pression artérielle pulsée aortique à partir de vitesses de propagation de l'onde de pouls déterminées.

Une installation de mesure de la vitesse de propagation de l'onde de pouls entre deux points du corps est actuellement connue. Celle-ci met en oeuvre un procédé de détermination du décalage temporel entre les instants de passage d'une même onde de pouls en deux points de mesure distincts du réseau artériel d'un être humain. Ces points de mesure sont disposés par exemple sur l'artère carotide primitive au niveau du cou et sur l'artère fémorale, au niveau de l'aine. Ce dernier capteur ou un capteur supplémentaire peut également être disposé sur l'artère brachiale à l'extrémité distale de l'avant-bras.

Pour déterminer la vitesse de propagation de l'onde de pouls, la distance séparant les deux points de mesure est mesurée manuellement à l'aide d'un mètre à ruban.

A partir de la distance séparant les deux points et du décalage temporel déterminé, la vitesse de propagation est estimée par simple division de la distance par le décalage temporel, en tenant compte toutefois de la distance séparant le coeur de l'un des points de mesure ou d'un éventuel trajet effectué en une direction opposée par l'une des deux ondes par rapport à l'autre, cette distance devant être déduite de la distance séparant les deux points de mesure.

Pour la détermination du décalage temporel entre les instants de passage de ta même onde de pouls en deux points distincts du réseau artériel, le procédé connu consiste à enregistrer l'évolution au cours du temps de la pression artérielle, à chaque point de mesure, puis à déduire, par analyse des deux enregistrements le décalage temporel entre les instants de passage de la même onde de pouls.

A cet effet, les courbes obtenues à partir de chaque enregistrement sont analysées. Ces courbes présentent, lors du passage de chaque onde de pouls un pic caractéristique représentatif d'une pulsation cardiaque.

Selon le procédé actuel, afin de déterminer le décalage temporel, on considère les maxima de pression de pics correspondant à une même onde de pouls, et on détermine le décalage temporel entre ces deux maxima. Ce décalage peut être obtenu manuellement par mesure directe sur des courbes imprimées représentatives des deux enregistrements ou par traitement informatique des deux enregistrements.

Toutefois, la détermination des maxima des pics est relativement délicate et variable selon plusieurs paramètres cliniques et biologiques. Ainsi, la précision de détermination des instants permettant de calculer le décalage temporel est faible, ce qui nuit à la qualité d'ensemble de la mesure.

L'invention a pour but de fournir un procédé de précision accrue, notamment en améliorant la détermination des instants permettant le calcul du décalage temporel entre les passages d'une même onde de pouls en deux points distants du réseau artériel d'un être vivant.

A cet effet, l'invention a pour objet un procédé de détermination du décalage temporel entre les instants de passage d'une même onde de pouls en deux points de mesure distincts d'un réseau artériel d'un être vivant, comportant les étapes de :

a) placer un capteur d'un paramètre représentatif de l'onde de pouls à chaque point de mesure du réseau artériel ;
b) enregistrer l'évolution au cours du temps du paramètre représentatif de l'onde de pouls à chaque point de mesure ;
c) déduire des deux enregistrements ledit décalage temporel, caractérisé en ce que l'étape de déduction du décalage temporel comporte les étapes de :
c1) déterminer, pour un premier enregistrement, un premier instant d'apparition de l'onde de pouls en identifiant le pied de l'onde de pouls,
c2) déterminer, pour le deuxième enregistrement, un deuxième instant d'apparition de la même onde de pouls en identifiant, dans le deuxième enregistrement, l'apparition de la partie correspondante de l'onde de pouls,
c3) calculer le décalage temporel entre les instants déterminés sur les premier et deuxième enregistrements.

Suivant des modes particuliers de mise en oeuvre, le procédé comporte l'une ou plusieurs des caractéristiques suivantes :

- le premier instant d'apparition du pied de l'onde de pouls est déterminé comme l'instant où la dérivée de la fonction enregistrée est maximale ;
- le deuxième instant d'apparition de la même onde de pouls sur le deuxième enregistrement est obtenu par recherche, dans le deuxième enregistrement, de l'instant où un coefficient de corrélation est maximal entre une plage du premier enregistrement définie autour du premier instant et une plage correspondante du deuxième enregistrement ;
- ledit coefficient de corrélation est un coefficient de corrélation linéaire ; et
- ledit paramètre représentatif de l'onde de pouls est la pression artérielle.

L'invention a en outre pour objet un procédé de détermination de la vitesse de propagation d'une onde de pouls entre deux points de mesure distants d'un réseau artériel d'un être vivant, comportant les étapes de :

A) mesurer la distance séparant les deux points de mesure sur l'être vivant,
B) déterminer le décalage temporel entre les instants de passage d'une même onde de pouls aux deux points de mesure ;
C) calculer la vitesse de propagation à partir de la distance séparant les deux points de mesure et du décalage temporel, caractérisé en ce que le décalage temporel entre les instants de passage de l'onde de pouls aux deux points de mesure est déterminé par un procédé selon l'une quelconque des revendications précédentes.

L'invention a enfin pour objet un procédé d'estimation de la pression pulsée aortique, caractérisé en ce qu'il comporte les étapes de :

a) déterminer la vitesse de propagation aortique d'une onde de pouls entre un point de l'artère carotide et un point de l'artère fémorale par mise en oeuvre d'un procédé tel que décrit ci-dessus,
b) déterminer la vitesse de propagation brachiale d'une onde de pouls entre un point de l'artère carotide et un point de l'artère brachiale, par mise en oeuvre d'un procédé tel que décrit ci-dessus,
c) mesurer la pression pulsée brachiale ;
d) estimer la pression aortique à partir des vitesses de propagation brachiale et aortique et de la pression pulsée brachiale.

L'invention sera mieux comprise à la lecture de la description qui va suivre, donnée uniquement à titre d'exemple et faite en se référant aux dessins, sur lesquels :

- La figure 1 est une vue schématique de l'installation selon l'invention, montrant les points d'implantation des capteurs sur un être humain ;
- la figure 2 est un organigramme explicitant les étapes du procédé selon l'invention ; et
- La figure 3 est une vue d'un enregistrement au cours du temps des mesures de pressions effectuées aux trois points de mesure du réseau artériel d'un être humain.

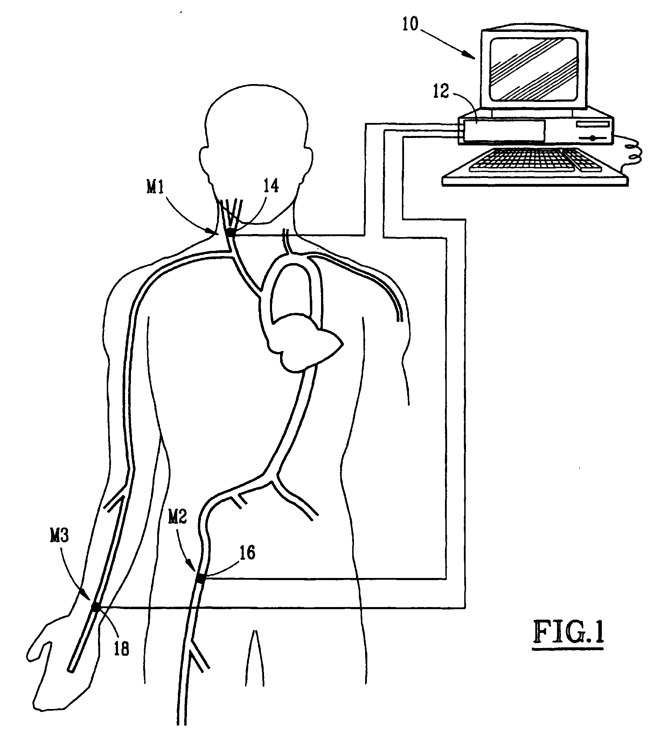

L'installation représentée sur la figure 1 est adaptée pour le calcul, d'une part, de la pression pulsée aortique d'un être humain et, d'autre part, de la vitesse de propagation d'une onde de pouls entre deux points de mesure distants du réseau artériel de l'être humain.

A cet effet, elle met en oeuvre un procédé de détermination du décalage temporel entre les instants de passage d'une même onde de pouls en deux points de mesure distincts du réseau artériel de l'être humain.

L'installation selon l'invention, représentée sur la figure 1, comporte une unité centrale de traitement d'informations 10, telle qu'un microordinateur. Elle comporte de plus une carte d'acquisition 12 assurant la liaison de trois capteurs de pression artérielle 14, 16, 18 à l'unité centrale de traitement d'informations 10. Elle comporte en outre une pédale pour arrêter l'acquisition et démarrer le traitement des données recueillies.

Le premier capteur de pression 14 est destiné à être placé sur le cou du patient, au-dessus de l'artère carotide primitive au point M1.

Le second capteur 16 est adapté pour être placé sur l'aine du patient au-dessus de l'artère fémorale au point M2.

Le troisième capteur 18 est adapté pour être placé sur l'extrémité distale de l'avant-bras du patient, au-dessus de l'artère radiale au point M3.

L'emplacement des capteurs peut être modifié selon le souhait de l'investigateur. Ainsi, il pourrait placer le capteur 14 en regard de l'artère brachiale et le capteur 18 en regard de l'artère radiale pour l'étude du segment artériel : brachial-radial.

Ces trois capteurs sont tenus sur le corps du patient en regard de l'artère correspondante par un bracelet élastique ou tout autre système de fixation adapté.

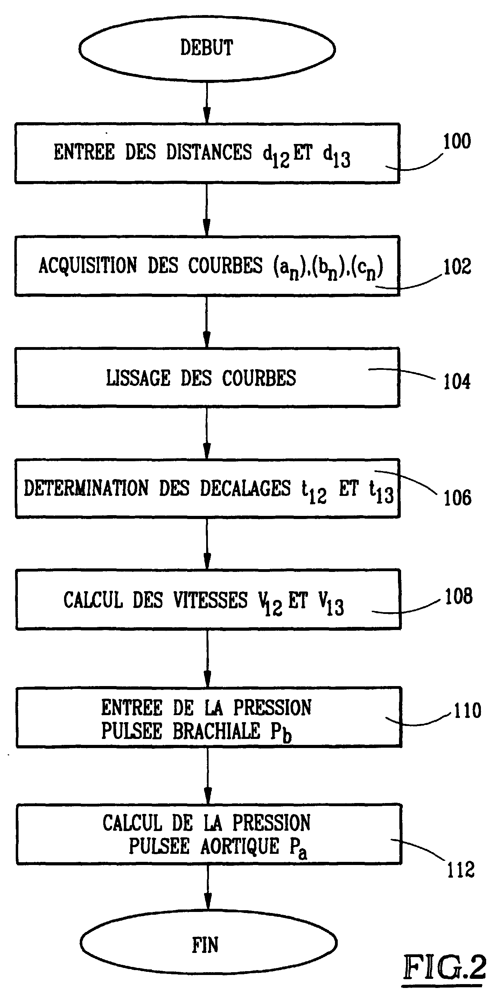

L'unité centrale de traitement d'informations 10 est munie d'une base de temps constituée par exemple par son horloge interne. Elle met en oeuvre un programme adapté dont l'organigramme est représenté sur la figure 2.

La première étape, notée 100, consiste à entrer dans l'unité centrale 10, la distance séparant les capteurs 14 et 16 d'une part, et 14 et 18 d'autre part. Ces distances sont notées $d_{12}$ et $d_{13}$ respectivement.

Elles sont mesurées par exemple à l'aide d'un mètre à ruban maintenu manuellement le long du corps du patient.

A l'étape 102, l'unité centrale de traitement d'informations 10 procède, sur un intervalle de temps déterminé, à l'enregistrement simultané de l'évolution de la pression artérielle aux points de mesure $M_1$, $M_2$, $M_3$, à partir des capteurs 14, 16, 18 et de la carte d'acquisition 12. A cet effet, l'unité centrale 10 enregistre, par exemple sur un disque dur, trois séries de mesures discrètes notées $(a_n)$, $(b_n)$, $(c_n)$ effectuées à des instants d'acquisition espacés d'un intervalle constant propre à la carte d'acquisition 12.

Après acquisition des enregistrements $(a_n)$, $(b_n)$ et $(c_n)$, chaque enregistrement est lissé à l'étape 104 afin de filtrer les hautes fréquences typiques des mesures physique. A cet effet, l'algorithme suivant est utilisé pour chaque enregistrement. Il est présenté ici pour l'enregistrement $(a_n)$.

```
for    i := 2 à N-1 do
       moyenne := (a[i-1]+a[i]+a[i+1])/3 ;
       d1 := abs(a[i-1]-moyenne) ;
       d2 := abs(a[i]-moyenne) ;
       d3 := abs(a[i+1]-moyenne) ;
       if d1 < d2 then a[i] := a[i-1] ; fi ;
       if d3 < d2 then a[i] := a[i+1] ; fi ;
od ;
```

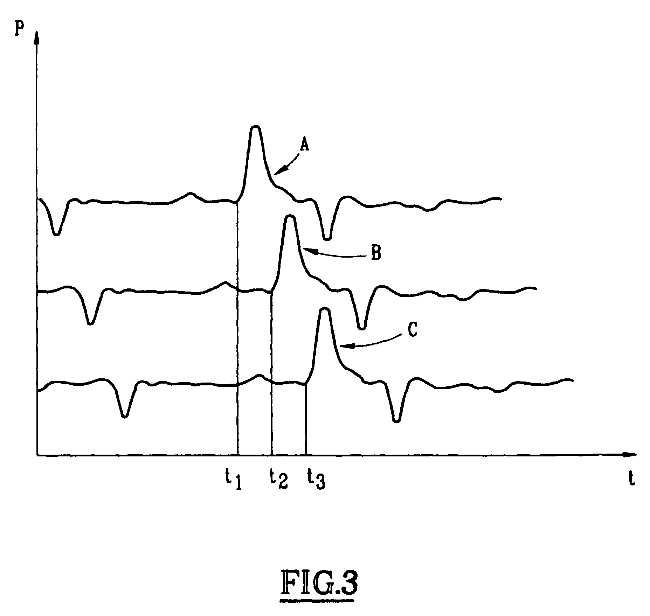

Après filtrage, les séries de mesures $(a_n)$, $(b_n)$, $(c_n)$ permettent la construction de courbes telles qu'illustrées sur la figure 3. Ces courbes, notées respectivement A, B, C, représentent l'évolution de l'onde de pression au cours du temps aux points $M_1$, $M_2$, $M_3$.

On constate sur ces courbes que l'onde de pouls passe à chacun des points de mesure avec un décalage temporel.

A l'étape 106, l'unité centrale 10 détermine, à partir de l'analyse des enregistrements $(a_n)$ et $(b_n)$ d'une part et $(a_n)$ et $(c_n)$ d'autre part, les décalages temporels entre les instants de passage d'une même onde de pouls aux points de mesure $M_1$ et $M_2$, d'une part, et $M_1$ et $M_3$ d'autre part. Ces décalages temporels sont notés $t_{12}$, $t_{13}$, respectivement.

Le même algorithme est mis en oeuvre pour le calcul du décalage $t_{12}$ et pour le calcul du décalage $t_{13}$. Aussi, seul le procédé de mesure du décalage temporel $t_{12}$ sera décrit en détail.

Pour la détermination du décalage temporel $t_{12}$, l'unité centrale 10 détermine d'abord pour le premier enregistrement $(a_n)$ l'instant $t_1$ d'apparition de l'onde de pouls en identifiant le pied de l'onde de pouls. Elle détermine ensuite pour le deuxième enregistrement $(b_n)$, l'instant $t_2$ d'apparition de la même onde de pouls en identifiant, dans le deuxième enregistrement $(b_n)$, l'apparition de la partie correspondante du pied de l'onde de pouls. Elle calcule enfin le décalage temporel à partir des instants $t_1$ et $t_2$ déterminés sur les premier et deuxième enregistrements.

L'instant $t_1$ d'apparition de l'onde de pouls sur le premier enregistrement $(a_n)$ est déterminé avantageusement comme l'instant où la dérivée de la fonction enregistrée est maximale.

L'instant $t_2$ d'apparition de l'onde de pouls correspondante au point $M_2$ est obtenu par recherche dans le deuxième enregistrement $(b_n)$ de l'instant où le coefficient de corrélation linéaire est maximal entre une plage du premier enregistrement définie autour de l'instant $t_1$ et une plage similaire correspondante du deuxième enregistrement. Cela permet de déterminer l'instant où la dérivée de la fonction enregistrée est maximale.

Plus précisément, la détermination du décalage temporel $t_{12}$ est réalisée par mise en oeuvre des étapes suivantes.

On calcule d'abord en utilisant un schéma de différences finies d'ordre 1 la valeur maximale en valeur absolue de la "dérivée" de la première courbe établie à partir de l'enregistrement $(a_n)$, i.e. la valeur C définie par

$$C = \{n \in \mathbb{IN} \text{ tel que } \forall_i \in [0,N-1] \ |a_{i+1} - a_i| \leq |a_{n+1} - a_n|\}$$

C correspond donc à l'indice de la suite où la différence entre deux éléments successifs est maximale.

On définit ensuite une fenêtre de travail [$m_0$, $M_0$] centrée en C en posant

$$m_0 = C - Valeur\ Min,$$

$$M_0 = C + Valeur\ Max\ ;$$

On cherche alors la valeur $K \in [O, N - M_0]$ qui maximise le coefficient de corrélation linéaire entre les courbes discrètes $a = (a_n)_{m0 \leq n \leq M0}$ et $b_k = (b_{n+k})_{m0 \leq n \leq M0}$, i.e. l'entier K de [0, N - $M_0$] qui est défini par

$$\frac{Covar\ (a,\ b_K)}{\sigma(a)\sigma(b_K)} = max\ (\ \frac{Covar\ (a,\ b_k)}{\sigma(a)\sigma(b_k)}\ ).$$

Ainsi, la recherche du coefficient de corrélation linéaire maximal s'effectue par translation progressive.

On détermine enfin l'instant $t_2$ correspondant à l'instant où a été effectuée la mesure d'indice K dans le deuxième enregistrement. Le décalage $t_{12}$ est alors calculé par la relation $t_{12} = t_2 - t_1$.

A l'étape 108, l'unité centrale 10 calcule, les vitesses $v_{12}$ et $v_{13}$ de propagation de l'onde de pouls, d'une part, entre les points $M_1$ et $M_2$ et d'autre part, entre les points $M_1$ et $M_3$.

La vitesse $v_{12}$ correspond à la vitesse de propagation aortique encore désignée par vitesse de propagation tronculaire. La vitesse $v_{13}$ correspond à la vitesse de propagation brachiale.

Le calcul des vitesses est effectué à partir des distances $d_{12}$ et $d_{13}$ mesurées, ainsi que des décalages temporels $t_{12}$ et $t_{13}$ déterminés à l'étape 106.

L'onde de pouls étant issue du coeur, il convient de corriger les distances $d_{12}$ et $d_{13}$ par la soustraction de la distance parcourue dans un sens opposé par l'une des deux ondes qui parcourt le trajet artériel dans un sens opposé.

Ainsi, à l'étape 108, les vitesses de propagation de l'onde de pouls sont calculées par les relations suivantes :

$$v_{12} = \frac{d_{12} - d}{t_{12}} \quad et \quad v_{13} = \frac{d_{13} - d'}{t_{13}}$$

où d et d' représentent les longueurs du trajet artériel parcouru par l'onde dans des sens opposés après rencontre d'une bifurcation conduisant celle-ci vers chacun des points de mesure. Par exemple, la distance d mesurée entre la carotide et l'aorte est soustraite de la distance mesurée entre la carotide et la fémorale puisque l'onde entre l'aorte et la carotide se propage dans un sens opposé (ascendant) à celui entre l'aorte et la fémorale (descendant).

Après calcul, l'unité centrale 10 met à disposition du praticien, notamment par affichage à l'écran, les valeurs $v_{12}$ et $v_{13}$.

Aux étapes 110 et 112, l'unité centrale procède à une estimation de la pression pulsée aortique.

Pour l'estimation de la pression pulsée aortique, le praticien possède d'abord manuellement à la mesure de la pression pulsée brachiale à l'aide d'un brassard gonflable et d'un stéthoscope appliqué de manière classique autour du bras du patient. La pression pulsée brachiale, encore appelée pression différentielle brachiale, est obtenue par différence de la pression systolique et de la pression diastolique mesurées par le praticien. Cette pression pulsée brachiale pourrait être aussi déterminée par des appareils automatiques de mesure de la pression artérielle (appareil électronique).

A l'étape 110, la pression pulsée brachiale ainsi obtenue est entrée dans l'unité centrale de traitement d'informations 10.

A l'étape 112, l'unité de traitement d'informations calcule la pression pulsée aortique estimée, notée $P_a$, à partir de la relation

$$P_a = P_b\ \sqrt{V_a/V_b}$$

où :

$V_a$ = vitesse de propagation aortique

$V_b$ = vitesse de propagation brachiale, et

$P_b$ = pression pulsée brachiale.

Les vitesses $v_a$ et $v_b$ sont prises égales respectivement aux vitesses $v_{12}$ et $v_{13}$ calculées à l'étape 108.

La pression pulsée aortique ainsi calculée donne une estimation satisfaisante de la valeur réelle de la pression pulsée aortique. Cette dernière est difficilement mesurable puisque l'aorte est inaccessible depuis l'extérieur du corps du patient.

Pour comprendre que l'estimation de la pression pulsée aortique est satisfaisante, il est possible de considérer le modèle d'une artère selon lequel, en l'absence de réflexion, le rapport de l'amplitude de la pression proximale $P_p$, à l'amplitude de la pression distale $P_d$ aux deux extrémités d'une artère est proportionnel à la racine carrée des caractéristiques d'impédance de l'artère, aux deux extrémités d'une artère, d'où la relation :

$$P_p/P_d = \sqrt{Z_p/Z_d}$$

où :

$Z_p$ est l'impédance proximale de l'artère, et

$Z_d$ est l'impédance distale de l'artère.

En outre, d'après la formule de "Water Hammer", selon laquelle la vitesse d'une onde pulsée est égale au produit de l'impédance caractéristique d'une artère par la densité du sang, l'expression précédente devient :

$$P_p/P_d = \sqrt{V_p/V_d}$$

où :

$V_p$ est la vitesse de propagation proximale de l'onde de pouls dans le système artériel, et

$V_d$ est la vitesse de propagation distale de l'onde de pouls dans le système artériel.

Ainsi, on comprend que du fait de la non uniformité de l'élasticité artérielle mesurée sur un être vivant, il est possible, à partir de la pression pulsée brachiale, de déterminer une estimation satisfaisante de la pression pulsée aortique.

On constate qu'avec le procédé mis en oeuvre pour la détermination des décalages temporels $t_{12}$ et $t_{13}$, la précision des mesures est accrue. En effet, l'utilisation du pied du pic traduisant l'onde de pouls sur les courbes obtenues à partir des enregistrements effectués aux différents points de mesure permet, en étant associée à une analyse de corrélation entre les différentes courbes, une estimation précise du décalage temporel.

Dans le procédé décrit ici, la détection du passage d'une onde de pouls en un point donné du réseau artériel est réalisée par suivi de l'évolution de la pression en ce point. Toutefois, tout autre paramètre représentatif de l'onde de pouls peut être utilisé, tel que le diamètre de l'artère ou la vitesse du flux sanguin. Un capteur adapté est alors utilisé en chaque point de mesure à la place du capteur de pression.

**Revendications**

1. Procédé de détermination du décalage temporel entre les instants de passage d'une même onde de pouls en deux points de mesure distincts ($M_1$, $M_2$) d'un réseau artériel d'un être vivant, comportant les étapes de :

   a) placer un capteur d'un paramètre représentatif de ronde de pouls (14,16) à chaque point de mesure du réseau artériel ;

   b) enregistrer l'évolution au cours du temps du paramètre représentatif de l'onde de pouls à chaque point de mesure ;

   c) déduire des deux enregistrements ledit décalage temporel, caractérisé en ce que l'étape de déduction du décalage temporel comporte les étapes de :

   c1) déterminer, pour un premier enregistrement, un premier instant ($t_1$) d'apparition de l'onde de pouls en identifiant le pied de l'onde de pouls,

   c2) déterminer, pour le deuxième enregistrement, un deuxième instant ($t_2$) d'apparition de la même onde de pouls en identifiant, dans le deuxième enregistrement, l'apparition de la partie correspondante de l'onde de pouls,

c3) calculer le décalage temporel entre les instants $t_1$ et $t_2$ déterminés sur les premier et deuxième enregistrements, et en ce que le deuxième instant ($t_2$) d'apparition de la même onde de pouls sur le deuxième enregistrement est obtenu par recherche, dans le deuxième enregistrement, de l'instant où un coefficient de corrélation est maximal entre une plage du premier enregistrement définie autour du premier instant ($t_1$) et une plage correspondante du deuxième enregistrement.

2. Procédé selon la revendication 1, caractérisé en ce que le premier instant ($t_1$) d'apparition du pied de l'onde de pouls est déterminé comme l'instant où la dérivée de la fonction enregistrée est maximale.

3. Procédé selon la revendication 2, caractérisé en ce que ledit coefficient de corrélation est un coefficient de corrélation linéaire.

4. Procédé selon l'une quelconque des revendications précédentes, caractérisé en ce que ledit paramètre représentatif de l'onde de pouls est la pression artérielle.

5. Procédé de détermination de la vitesse de propagation d'une onde de pouls entre deux points de mesure distants d'un réseau artériel d'un être vivant, comportant tes étapes de :

   A) mesurer la distance séparant les deux points de mesure sur l'être vivant,
   B) déterminer le décalage temporel entre les instants de passage d'une même onde de pouls aux deux points de mesure ;
   C) calculer la vitesse de propagation à partir de la distance séparant les deux points de mesure et du décalage temporel, caractérisé en ce que te décalage temporel entre les instants de passage de l'onde de pouls aux deux points de mesure est déterminé par un procédé selon l'une quelconque des revendications précédentes.

6. Procédé d'estimation de la pression pulsée aortique, caractérisé en ce qu'il comporte les étapes de :

   a) déterminer ta vitesse de propagation aortique d'une onde de pouls entre un point ($M_1$) de l'artère carotide et un point ($M_2$) de l'artère fémorale par mise en oeuvre d'un procédé selon la revendication 6,
   b) déterminer la vitesse de propagation brachiale d'une onde de pouls entre un point ($M_1$) de l'artère carotide et un point ($M_3$) de l'artère brachiale, par mise en oeuvre d'un procédé selon la revendication 6,
   c) mesurer la pression pulsée brachiale ;
   d) estimer la pression aortique à partir des vitesses de propagation brachiale et aortique et de la pression pulsée brachiale.

7. Procédé selon la revendication 6, caractérisé en ce que la pression pulsée aortique $P_a$ est déterminée par la relation :

$$P_a = P_b \sqrt{V_a / V_b}$$

où :

   $V_a$ est la vitesse de propagation aortique
   $V_b$ est la vitesse de propagation brachiale, et
   $P_b$ est la pression pulsée brachiale.

FIG.1

```
                    ┌──────────────┐
                    │    DEBUT     │
                    └──────────────┘
                           │
                           ▼
        ┌────────────────────────────────────┐
        │  ENTREE  DES  DISTANCES  d₁₂ ET d₁₃ │────── 100
        └────────────────────────────────────┘
                           │
                           ▼
        ┌────────────────────────────────────┐
        │ ACQUISITION  DES  COURBES (aₙ),(bₙ),(cₙ) │─── 102
        └────────────────────────────────────┘
                           │
                           ▼
        ┌────────────────────────────────────┐
        │       LISSAGE  DES  COURBES        │────── 104
        └────────────────────────────────────┘
                           │
                           ▼
        ┌────────────────────────────────────┐
        │ DETERMINATION  DES  DECALAGES t₁₂ ET t₁₃ │── 106
        └────────────────────────────────────┘
                           │
                           ▼
        ┌────────────────────────────────────┐
        │  CALCUL  DES  VITESSES  V₁₂ ET V₁₃ │────── 108
        └────────────────────────────────────┘
                           │
                           ▼
        ┌────────────────────────────────────┐
        │         ENTREE  DE  LA  PRESSION   │────── 110
        │         PULSEE  BRACHIALE  Pb      │
        └────────────────────────────────────┘
                           │
                           ▼
        ┌────────────────────────────────────┐
        │        CALCUL  DE  LA  PRESSION    │
        │        PULSEE  AORTIQUE  Pa        │────── 112
        └────────────────────────────────────┘
                           │
                           ▼
                    ┌──────────────┐
                    │     FIN      │
                    └──────────────┘
```

Début

ENTREE DES DISTANCES $d_{12}$ ET $d_{13}$ — 100

ACQUISITION DES COURBES $(a_n),(b_n),(c_n)$ — 102

LISSAGE DES COURBES — 104

DETERMINATION DES DECALAGES $t_{12}$ ET $t_{13}$ — 106

CALCUL DES VITESSES $V_{12}$ ET $V_{13}$ — 108

ENTREE DE LA PRESSION PULSEE BRACHIALE $P_b$ — 110

CALCUL DE LA PRESSION PULSEE AORTIQUE $P_a$ — 112

FIN

**FIG.2**

FIG.3

**Office européen des brevets**

# RAPPORT DE RECHERCHE EUROPEENNE

Numéro de la demande

EP 00 40 1432

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | ASMAR R ET AL.: "Assessment of Arterial Distensibility by Automatic Pulse Wave Velocity Measurement" HYPERTENSION, vol. 26, no. 3, 1995, pages 485-490, XP000949372 | 1,2,4,5 | A61B5/0285 A61B5/021 |
| A | * partie "PWV Measurement" * * partie "Statistical Analysis" * * partie "Conclusion" * | 3,6 | |
| X | BENTHIN M ET AL.: "Calculation of pulse wave velocity using cross correlation-effects of reflexes in the arterial tree" ULTRASOUND IN MEDICINE AND BIOLOGY, vol. 17, no. 5, 1991, pages 461-469, XP000949395 usa * partie "Calculation of PWV using cross correlation" * * Results * | 1,4,5 | |
| | | | **DOMAINES TECHNIQUES RECHERCHES** (Int.Cl.7) |
| A | RAMSEY M W ET AL: "REAL-TIME MEASUREMENT OF PULSE WAVE VELOCITY FROM ARTERIAL PRESSURE WAVEFORMS" MEDICAL AND BIOLOGICAL ENGINEERING AND COMPUTING,GB,PETER PEREGRINUS LTD. STEVENAGE, vol. 33, no. 5, 1 septembre 1995 (1995-09-01), pages 636-642, XP000530850 ISSN: 0140-0118 * partie "3 Calculation of pulse wave velocity" * * page 641 * | 1-5 | A61B |

-/--

Le présent rapport a été établi pour toutes les revendications

| Lieu de la recherche | Date d'achèvement de la recherche | Examinateur |
|---|---|---|
| LA HAYE | 22 septembre 2000 | Knüpling, M |

Office européen
des brevets

**RAPPORT DE RECHERCHE EUROPEENNE**

Numéro de la demande

EP 00 40 1432

## DOCUMENTS CONSIDERES COMME PERTINENTS

| Catégorie | Citation du document avec indication. en cas de besoin, des parties pertinentes | Revendication concernée | CLASSEMENT DE LA DEMANDE (Int.Cl.7) |
|---|---|---|---|
| A | DE 845 379 C (KLEIN DR. PAUL) 12 juillet 1951 (1951-07-12) * page 2, ligne 1 - ligne 12 * * page 2, ligne 59 - ligne 75 * | 1,2,4,5 | |
| A | DE 704 186 C (ELMQUIST DR. RUNE, PETERSON DR. PETER) 20 février 1941 (1941-02-20) * page 1, ligne 1 - ligne 31 * | 1,4,5 | |
| A | FR 2 481 917 A (INST NAT SANTE RECH MED) 13 novembre 1981 (1981-11-13) * page 7, ligne 16 - ligne 35 * * page 8, ligne 33 - page 9, ligne 3 * * page 11, ligne 3 - ligne 13 * | 1,4,5 | |
| A | US 3 090 377 A (SALISBURY P F, WICHMANN T F) 21 mai 1963 (1963-05-21) * colonne 3, ligne 42 - colonne 4, ligne 23 * | 1,5-7 | |

**DOMAINES TECHNIQUES RECHERCHES (Int.Cl.7)**

Le présent rapport a été établi pour toutes les revendications

| Lieu de la recherche | Date d'achèvement de la recherche | Examinateur |
|---|---|---|
| LA HAYE | 22 septembre 2000 | Knüpling, M |

CATEGORIE DES DOCUMENTS CITES

X : particulièrement pertinent à lui seul
Y : particulièrement pertinent en combinaison avec un
autre document de la même catégorie
A : arrière–plan technologique
O : divulgation non–écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant

## ANNEXE AU RAPPORT DE RECHERCHE EUROPEENNE
## RELATIF A LA DEMANDE DE BREVET EUROPEEN NO.

EP 00 40 1432

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

22-09-2000

| Document brevet cité au rapport de recherche | Date de publication | Membre(s) de la famille de brevet(s) | Date de publication |
|---|---|---|---|
| DE 845379 C | | AUCUN | |
| DE 704186 C | | AUCUN | |
| FR 2481917 A | 13-11-1981 | AUCUN | |
| US 3090377 A | 21-05-1963 | AUCUN | |

Pour tout renseignement concernant cette annexe : voir Journal Officiel de l'Office européen des brevets, No.12/82